# NEW EUROPEAN PATENT SPECIFICATION

(11) **EP 0 593 792 B2**
(45) Date of publication and mention of the opposition decision: **07.01.2004**
(45) Mention of the grant of the patent: 14.05.1997
(21) Application number: 92117551.9
(22) Date of filing: 14.10.1992
(51) Int. Cl.: C12N 15/52, C12P 13/08, C12N 1/19, C12N 1/21

(54) **Novel L-threonine-producing microbacteria and a method for the production of L-threonine**
Neuartiges L-threoninproduzierendes Mikrobakterium und eine Herstellungsmethode für L-Threonin
Nouvelle micro-bactérie capable de produire de la L-thréonine et procédé de production de la L-thréonine

(43) Date of publication of application: 27.04.1994
(73) Proprietor: Ajinomoto Co., Inc., Tokyo 104 (JP)
(72) Inventor: Debabov, Vladimir Georgievich VNHGENETIKA, Moscow 113545 (SU); Kozlov, Uri Ivanovich VNHGENETIKA, Moscow 113545 (SU); Khurges, Evgenyi Moiseevich VNHGENTIKA, Moscow 113545 (SU); Lifshits, Vytalyi Arkadievich VNHGENETIKA, Moscow 113545 (SU); Zhdanova, Nelli Isaakovna VNHGENTIKA, Moscow 113545 (SU); Gusyatiner, Michail Markovich VMHGENETIKA, Moscow 113545 (SU); Sokolov, Alexander Konstantinovich VNHGENTIKA, Moscow 113545 (SU); Bachina, Tatiana Alexandrovna VNHGENETIKA, Moscow 113545 (SU); Chistoserdov, Andrei Yurevich VNHGENETIKA, Moscow 113545 (SU); Tsigankov, Uri Dmitrievich VNHGENETIKA, Moscow 113545 (SU); Yankovsky, Nikolai Kazimirovich VNHGENETIKA, Moscow 113545 (SU); Mashko, Sergei Vladimirovich VNHGENETIKA, Moscow 113545 (SU); Lapidus, Alla Lvovna VNHGENETIKA, Moscow 113545 (SU); Gavrilova, Oksana Fedorovna VNHGENETIKA, Moscow113545 (SU); Rodionov, Oleg Alexandrovich VNHGENETIKA, Moscow 113545 (SU)
(74) Representative: Strehl Schübel-Hopf & Partner

(56) References cited:
- FR-A- 2 640 640
- GB-A- 2 208 866
- US-A- 4 278 765

## Description

### BACKGROUND OF THE INVENTION

### Field of the Invention

The present invention is concerned with a novel microorganism and microbiological process for the production of L-threonine. L-threonine is an essential amino acid, used in various nutrient and medical compositions. Moreover, threonine is an important additive to animal fodder, a valuable reagent used in the pharmaceutical and chemical industries, and a growth factor for amino acid-producing microorganisms used in the production of, for example, lysine and homoserine.

### Discussion of the Background

Prior to the present invention, natural strains of L-threonine-producing microorganisms and artificial mutants thereof have been used to fermentatively produce L-threonine. L-threonine-producing artificial mutants belonging to the genera *Escherichia, Serratia, Brevibacterium* and *Corynebacterium* are known, and most of them are resistant to α-amino-β-hydroxyvaleric acid. With respect to the genus Escherichia, methods for producing L-threonine using a strain transformed with a recombinant plasmid DNA comprising a threonine operon are shown in Japanese Laid-Open Patent Application Nos. 55-131397, 59-31691 and 56-15696, and in PCT Laid-Open Application No. 90-04636.

Primary carbon sources added to the fermentation media of these L-threonine-producing microorganisms include glucose, sucrose, starch hydrolysate and molasses. Based on (1) the level of threonine biosynthesis and (2) the expenditure coefficient, defined as the value (in g) of sugar necessary to produce 1 g of L-threonine, *E. coli* strain BKIIM B-3996 (hereafter "strain B-3996" or "B-3996") is the best of the strains disclosed (PCT Laid-Open Application No. 90-04636). Strain B-3996 can synthesize up to 85 g/l threonine, with an expenditure coefficient of 2 g sugar per 1 g of threonine, provided that a sugar-ammonium mixture is added to the nutrient medium (in response to a signal from the pH sensor used in a standard laboratory fermenter during cultivation).

However, most strains used to produce L-threonine, such as *E. coli* BKIIM B-3996, are L-isoleucine auxotrophic. Therefore, an enzyme in the pathway from L-threonine to L-isoleucine is defective. Accordingly, L-isoleucine must be added to cultures of L-threonine-producing strains. L-Isoleucine auxotrophy in L-threonine-producing strains prevents (1) by-production of isoleucine, thus decreasing L-threonine accumulation, and (2) surplus production of L-isoleucine, thus suppressing expression of the threonine operon(s) through the corresponding attenuator.

When using such an isoleucine-dependent strain, strict control of the amount of isoleucine added into a medium is required, because growth of the strain and threonine productivity must be moderately balanced. The more isoleucine added to the medium, the more the strain grows. However, threonine productivity is simultaneously repressed in an inverse manner.

From this point of view, strain B-3996 requires isoleucine (known as "leaky-type"), and as a result, exhibits low productivity of L-threonine in media containing molasses as both a carbon source and an energy source. Molasses is a non-nutrient raw material which is much cheaper than sucrose, but which also contains a high amount of amino acids (ex. isoleucine). Thus, although it is highly desired in the art to use molasses as both a carbon source and an energy source, the isoleucine present in molasses suppresses production of L-threonine in L-threonine-producing bacteria.

### SUMMARY OF THE INVENTION

Accordingly, one object of the present invention is to provide a novel microorganism producing a high concentration of threonine and a process for producing L-threonine using this microorganism. This object is accomplished by the subject matter of the appended claims. As procaryotic cells cells belonging to the genus Escherichia, Serratia, Brevibacterium or Corynebacterium are preferred. Cells belonging to the genus Escherichia coli are most preferred.

A further object of the present invention is to provide a novel method for the fermentative production of threonine, in which a microorganism produces a high concentration of threonine in a medium containing molasses as a raw material.
These and other objects will become apparent during the following detailed description of the preferred embodiments. The preferred microorganism belonging to the genus Escherichia coli produces a large number of generations and a large amount of L-threonine when cultured in a medium containing molasses as a raw material.

### BRIEF DESCRIPTION OF THE DRAWINGS

A more complete appreciation of the invention and many of the attendant advantages thereof will be readily obtained as the same becomes better understood by reference to the following detailed description when considered in connection with the accompanying drawings, wherein:
Figure 1 is a scheme for obtaining the plasmid pPR39;
Figure 2 is a scheme for obtaining the plasmid pPRT610:
Figure 3 is a scheme for obtaining the plasmid pPRT614:
Figure 4 graphically profiles the growth curve and accumulation of L-threonine when culturing B-3996 and B-5318, wherein ○ represents the growth curve of B-5318, □ represents the accumulation of L-threonine when culturing B-5318, ● represents the growth curve of B-3996, and ■ represents the accumulation of L-threonine when culturing B-3996.

### DETAILED DESCRIPTION OF THE PREFERRED EMBODIMENTS

The present inventors have achieved the goals of the present invention by providing the strain *E. coli* BKIIM B-5318, which produces up to 70 g/l of threonine during 32 hours of growth on a molasses medium.
In addition other suitable microorganisms, which may be used according to the present invention comprise eucaryotic cells belonging to the genus Saccharomyces, preferably to Saccharomyces cerevisiae, and procaryotic cells belonging to the genus Escherichia, Serratia, Brevibacterium or Corynebacterium. (The expenditure coefficient of this strain is 1.7 as defined above).

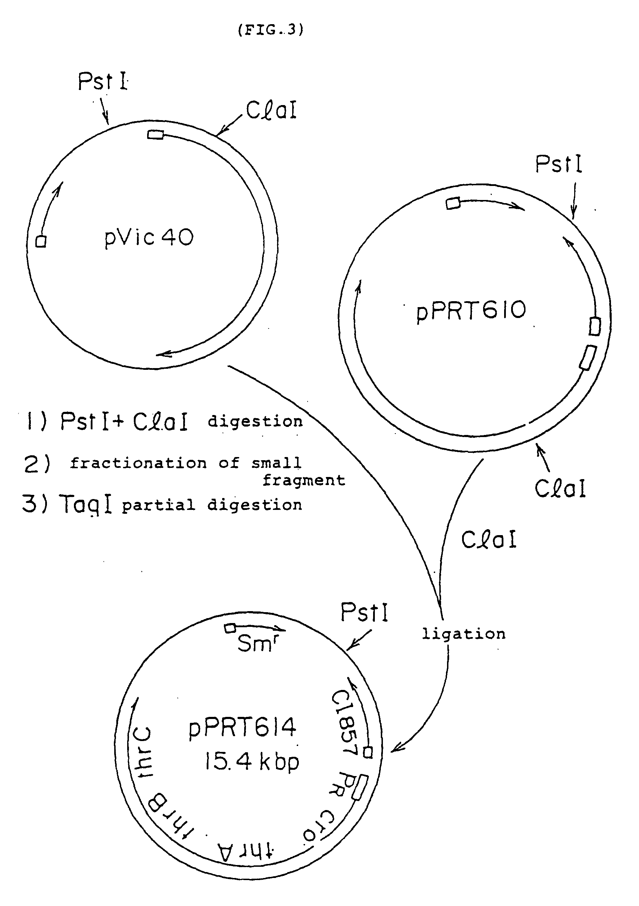

The novel strain *E. coli* BKIIM B-5318 was obtained by transformation of a new recipient strain *E. coli* VNII Genetika TDH-7 transformed with plasmid pPRT614. As shown in Fig. 3, plasmid pPRT614 was obtained by substituting the regulatory region of the threonine operon of plasmid pVIC40 for the PR promoter and temperature-sensitive lambda-phage cI repressor. The pVIC40 Taql-Clal fragment is introduced into pPRT610, which is missing the upstream region of the thrA gene, lost during its production. As a result, pPRT614 is provided with an entire thrA gene (Fig. 3).

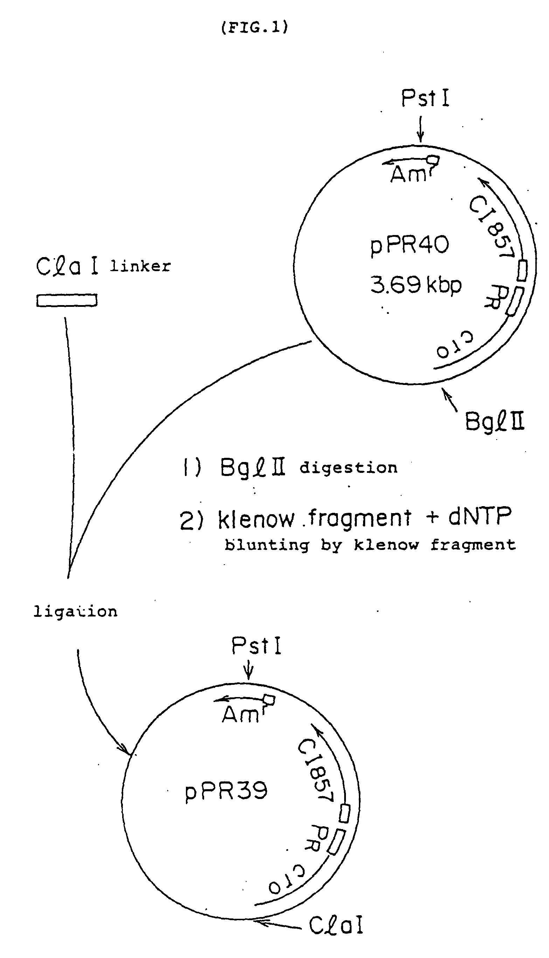

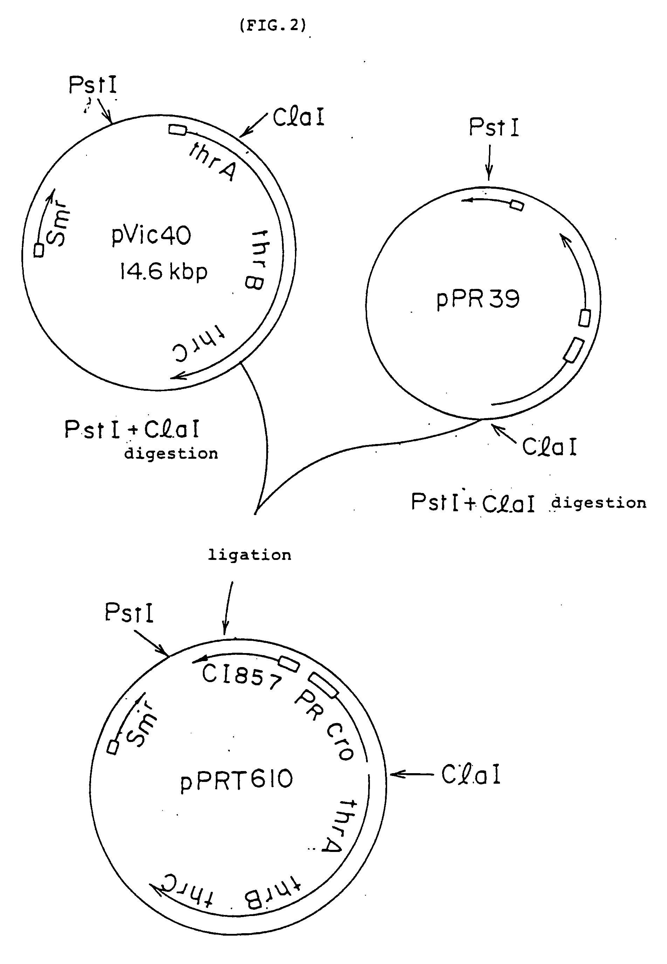

Plasmid pPR40 was the donor of the promoter and repressor (Figs. 1, 2). Figure 1 shows the introduction of the Clal site (chemically synthesized by known methods) into the BglII site of pPR40 to produce pPR39. Figure 2 shows digestion of both pVIC40 and pPR39 with both Pstl and Clal. By ligating the above fragments to locate the lambda-phage promoter and repressor upstream from the threonine operon, pPRT610 was prepared. However, the Clal site is located in the coding region of thrA gene in pVIC40.

The new recipient strain *E. coli* VNIIGenetika TDH-7 is a isoleucine prototroph. It was obtained from plasmidless cells of strain VNIIGenetika TDH-6. *E. coli* VNIIGenetika TDH-6 is deposited in the Research Institute of Genetics and Industrial Microorganism Breeding at Russia 113545 Moscow, 1 Dorozhny Proezd., 1 (Registration No. BKIIM B-3420).

For this purpose, the plasmidless cells of strain *E. coli* VNIIGenetika TDH-6 were infected by phage P1, earlier propagated on cells of strain *E. coli*c6000. Thereafter, transductants were selected which were capable of growth on isoleucine-free media. The selected strain was designated *E. coli* VNIIGenetika TDH-7. TDH-7 was then transformed with plasmid pPRT614, leading to the establishment of the strain *E. coli* BKIIM B-5318.

The new strain *E. coli* BKIIM B-5318 differs from the known strain by the fact that it is capable of growth on enriched nutrient media (for example, on media with molasses), which leads to a more effective production of threonine. The difference in threonine production results from two modifications:
(1) A temperature-sensitive lambda-phage cl repressor and PR promoter replaces the regulatory region of the threonine operon in plasmid pVIC40: and
(2) Strain B-5318 becomes prototrophic with regard to isoleucine.

*E. coli* BKIIM B-5318 produces more than 70 g/l of threonine during 32 hours of fermentation on molasses, in standard laboratory conditions, and at temperatures of 38-41°C.

The new producent of threonine *E. coli* BKMIIM B-5318 was deposited in the collection of microorganism cultures of the All-Union Research Institute of Antibiotics (Registration No. 2070) and also in the Research Institute of Genetics and Industrial Microorganism Breeding at Russia 113545 Moscow, 1 Dorozhny Proezd., 1 (Registration No. BKIIM B-5318).

The new strain *E. coli* BKIIM B-5318 has the following morphological and biochemical traits:
(1) Cell Morphology: Gram-negative: round-ended rods with low mobility, 1.5 - 2.0 MICROMETERS in length.
(2) characteristics of cultures:
   (A) Meat-Peptone Agar: After 24 hours of growth at 37°C cultures form rounded, whitish, semi-transparent colonies with a diameter of 1.5 - 3.0 mm: the surfaces of the colonies are smooth, the edges are even or slightly wavy, the center of the colony is elevated, the structure is homogeneous, and the consistency is paste-like and easy to emulgate.
   (B) Agar Luria: After 24 hours of growth at 37°C, cultures form whitish semi-transparent colonies 1.5 - 2.5 mm in diameter; the surface of the colonies is smooth, the edges are even, the structure homogeneous, and the consistency is paste-like and easily emulgated.
   (C) Agarized Adams Medium: After 40 - 48 hours of growth at 37°C, cultures form colonies 0.5 - 1.5 mm in diameter: greyish-white, semi-transparent, slightly elevated with a shining surface.
   (D) Growth on Meat-Peptone Broth: The specific rate of growth at 37°C is 1.3 h⁻¹; after 24 hours of growth, a drastic homogeneous turbidity and characteristic odor are observed.
(3) Physico-Biochemical Traits: Growth along a prick of the meat-peptone agar is even along the entire prick. The microorganism is a facultative anaerobe. It does not liquify gelatine. Grows well on milk and causes milk coagulation. Does not form indole. Resistant to streptomycin. It is resistant to the potential product inhibitors L-threonine and L-homoserine.

Temperature-sensitivity: Grows on meat-peptone broth at 43°C and lower. Optimal growth revealed at 37-38°C.

pH sensitivity: Grows on media at pH from 6.0 to 8.0; optimal pH = 7.0.

Growth on different carbon sources: Grows well on sucrose, glucose, lactose, mannose, galactose, xylose, glycerol, mannite with the formation of acid and gas.

Growth on different nitrogen sources: Assimilates nitrogen in the form of ammonia, nitrates, and also nitrogen of some organic compounds.

Plasmid content: The cells comprise a multi-copy hybrid plasmid pPRT614 (mol. mass 10.2 MD) that provides streptomycin resistance and carries the threonine operon genes, the lambda-phage promoter and the temperature-sensitive repressor cl gene.

The plasmid is highly stable in strain *E. coli* BKIIM B-5318, even during growth in the absence of selective pressure to maintain the plasmid. The stability of the strain characteristics provided by the plasmid were evaluated for strain *E*. *coli* BKIIM B-5318 (plasmid pPRT614) and for strain-prototype *E. coli* BKIIM B-3996 (plasmid pVIC40). The cells of both strains were grown in the presence of streptomycin until the onset of the early stationary stage. Cells were then seeded on Luria medium without antibiotic, at a starting titre 50. After 48 hours of cultivation at 36.7 - 40°C, the cells obtained were reseeded on Luria medium to the starting titre 50, and again cultivated for 48 hours at the same temperature. Each passage corresponded to 20 generations. After the indicated passages, the cells were seeded on Luria agar, and the colonies were checked for streptomycin resistance. In both strains, the proportion of plasmidless cells was less than 1%.

### Production of L-Threonine:

A culture of strain *E. coli* BKIIM B-5318 prepared as described above was grown on agarized Adams medium containing streptomycin. The suspension of cells was then seeded in liquid inoculum or fermentation medium, comprising sources of carbon, nitrogen, essential mineral salts and nutrient additives in the form of protein hydrolysates. (The presence of L-isoleucine in the fermentation medium is not necessary). The growth of the inoculum is carried out at pH 6.8 - 7.2 at a temperature of 36-38°C, with continuous aeration and stirring. The inoculum, or a suspension of cells washed off the agar, is used for inoculation of the fermentation medium.

The fermentation is carried out in fermenters equipped with a system for pH stabilization at pH 6.8 - 7.2 and at a temperature of 38 - 40°C, with continuous aeration and stirring. Aqueous ammonia, or carbon- and nitrogen-balanced molasses-ammonia nutrient additive are used as pH-stabilizing agents. The duration of fermentation depends on the inoculum dose and the level of growth factor enrichment of the fermentation medium, but generally varies from 24 to 60 hours.

The specific expense of carbon sources needed for the synthesis or 1 g of L-threonine (the expenditure coefficient) is 1.8 g. No accumulation of aminoacetone in the culture medium was observed.

Other features of the invention will become apparent in the course of the following descriptions of exemplary embodiments which are given for illustration of the invention and are not intended to be limiting thereof.

### Example 1

Cultures of strains *E. coli* BKIIM B-3996 (prototype) and *E. coli* BKIIM B-5318 were grown separately on agarized Adams medium containing sucrose (0.2%) and streptomycin (100 mg/ml) for two days, then suspended in a physiological solution. Inoculum medium (500 ml) was seeded with a sample of the suspension (10 ml: titre = 10⁸). The inoculum medium had the following composition (percentages are by weight): molasses - 3%, ammonium sulfate - 0.5%, dipotassium phosphate (K₂HPO₄) - 0.2%, magnesium sulfate heptahydrate (MgSO₄.7H₂O) - 0.04%, ferrous sulfate (FeSO₄.7H₂O) - 0.002%, manganese (II) sulfate (MnSO₆·5H₂O) - 0.002%, yeast autolysate - 0.2%, water - the remaining balance.

The inoculum is grown for 20 hours in a laboratory fermenter (vol. 0.7 1), with aeration (0.5 l/min) and stirring (1200 rotations/min) at a temperature of 39°C. The pH was maintained in the range 6.9 - 7.2 by an automatic inlet providing a molasses-ammonium nutrient additive (a mixture of ammonium water and molasses in a molar ratio 2.92:1, with a molasses concentration in the mixture of 300 g/l). Fermentation was carried out for 32 hours.

The results are given in Table 1 below:

**Table 1**

| Strain | Threonine concentration (g/l) | Expenditure coefficient (g sugar consumed g threonine produced) |
|---|---|---|
| B-3996 | 46.7 | 2.5 |
| B-5318 | 70.4 | 1.7 |
| Note: Accumulation of threonine and the expenditure coefficient for strains BKIIM B-3996 and BKIIM B-5318 after 32 hours fermentation using molasses. | | |

After 32 hours, *E. coli* BKIIM B-5318 produces 70.4 g/l L-threonine, whereas the prototype strain B-3996 produces only 46.7 g/l L-threonine. The corresponding expenditure coefficients are 1.7 and 2.5, respectively.

Thus, the present microorganism increases the output of L-threonine in fermentation media containing molasses in comparison to the prototype strain, while the expenditure coefficient is simultaneously lowered.

### Example 2

Strains *E. coli* BKIIM B-3996 (prototype) and *E. coli* BKIIM B-5318 were grown separately on agarized M9 medium containing 0.2% of sucrose and 100 mg/ml of streptomycin for 1 day, then suspended in an inoculum medium shown in Table 2. A sample of the suspension having a titre of 10⁸ (1 ml) was used to seed 250 ml of an inoculum medium prepared by dissolving the ingredients shown in Table 2 into 1 I of water.

**Table 2**

| | | |
|---|---|---|
| ammonium sulfate (NH₄)₂SO₄ | 5 (g/l) | 0.5 (%) |
| dipotassium phosphate (K₂HPO₄) | 2 | 0.2 |
| sodium chloride (NaCl) | 0.6 | 0.06 |
| magnesium sulfate (MgSO₄·7H₂O) | 0.4 | 0.04 |
| ferrous sulfate (FeSO₄·5H₂O) | 0.02 | 0.002 |
| manganese sulfate (MnSO₄·5H₂O) | 0.02 | 0.002 |
| yeast autolysate | 2 | 0.2 |
| sucrose (autoclaved separately) | 30 | 3.0 |

The inoculum was grown for 12 hours in a laboratory fermenter (volume = 1.0 l) with aeration (0.25 l/min) and stirring (700 rotations/min at first, then set to keep the oxygen pressure at a level of more than 2%) at a temperature of 39°C. The pH was maintained in the range of 6.7-7.1 by an automatic inlet providing ammonium gas. After fermentation, 25 ml of the culture was added to 250 ml of a fermentation medium. The contents of the fermentation medium are shown in Table 3.

**Table 3**

| | | |
|---|---|---|
| ammonium sulfate (NH₄)₂SO₄ | 4.5 (g/l) | 0.45 (%) |
| dipotassium phosphate (K₂HPO₄) | 1.8 | 0.18 |
| sodium chloride (NaCl) | 0.6 | 0.06 |
| magnesium sulfate (MgSO₄·7H₂O) | 0.36 | 0.036 |
| ferrous sulfate (FeSO₄·5H₂O) | 0.018 | 0.0018 |
| manganese sulfate (MnSO₄·5H₂O) | 0.018 | 0.0018 |
| yeasts autolysate | 1.8 | 0.18 |
| sucrose (autoclaved separately) | 27 | 2.7 |
| antifoam agent (autoclaved separately) | 1 ml/l | |

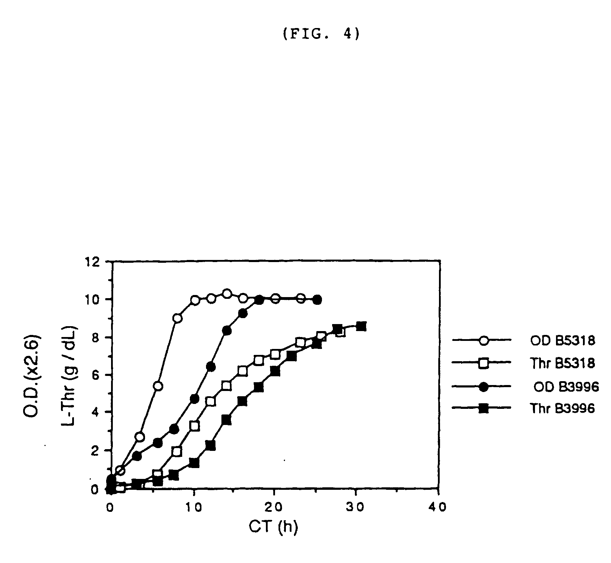

The inoculum in the fermentation medium was grown for 28 hours in a laboratory fermenter (volume = 1.0 l) with aeration (0.25 l/min) and stirring (700 rotations/min at first, then set to keep the oxygen pressure at a level of more than 2%) at a temperature or 39°C. The pH was maintained in the range of 6.7 - 7.1 by an automatic inlet providing ammonium gas. The sucrose concentration of the medium was maintained below 20 g/l by adding 600 g/l (60%) of an aqueous sucrose solution. The growth curve and L-threonine accumulation profiles are shown in Figure 4, the fermentation results are shown in Table 4.

**Table 4**

| strain | accumulation of L-threonine (g/dl) | yield to sugar (%) | expenditure coefficient (g sugar/g threonine) | culturing time (hr) |
|---|---|---|---|---|
| B-3669 | 8.3 | 40 | 2.4 | 30.0 |
| B-5318 | 8.2 | 41 | 2.4 | 28.0 |

As is apparent from Table 4. B-5318 is superior to B-3996 in growth speed. Thus, the culturing time can be shortened and contamination can be prevented.

## Claims

1. A cell of a procaryote transformed with a DNA sequence comprising:
(a) a first inducible operator sequence
(b) a second DNA sequence directly downstream the first DNA sequence, comprising a threonine operon that contains a region defective in attenuation, wherein the cell to be transformed is a isoleucine prototrophic cell.

2. A cell according to claim 1, wherein
the first inducible operator sequence comprises a lambda phage temperature sensitive cl repressor and a promotor.

3. A cell comprising a DNA sequence according to claim 1 or 2 in a vector capable of expressing said DNA sequence.

4. A cell according to claim 3, wherein the vector is a plasmid.

5. A cell according to claim 3 or 4, wherein the vector comprises a RSF 1010 replicator.

6. A cell according to claim 3, 4 or 5, wherein the vector comprises a selectable marker gene.

7. A cell according to claim 6, wherein said selective marker gene is a gene providing streptomycin resistance.

8. A cell according to claim 4, wherein said plasmid is pPRT614.

9. The cell of any of the claims 1 to 8, wherein the cell belongs to the genus Escherichia, Serratia, Brevibacterium or Corynebacterium.

10. The cell of claim 9, wherein the cell to be transformed is Escherichia coli VNIIGenetika TDH-7.

11. The cell of claim 9, which is Escherichia coli BKIIM B-5318.

12. A method for producing L-threonine, which comprises the steps of:
(a) culturing a cell according to any of the claims 1 to 11 in a medium;
(b) accumulating said L-threonine in the medium and the cell;
(c) collecting said L-threonine from the medium and the cell.

## Patentansprüche

1. Prokaryotische Zelle, die mit einer DNA-Sequenz, transformiert ist, die
(a) eine erste induzierbare Operatorsequenz und
(b) eine zweite DNA-Sequenz umfaßt, die unmittelbar stromabwärts von der ersten DNA-Sequenz liegt und ein Threoninoperon umfaßt, welches einen in der Attenuation fehlerhaften Bereich enthält, wobei die zu transformierende Zelle eine Isoleucinprototrophe Zelle ist.

2. Zelle gemäß Anspruch 1, wobei die erste induzierbare Operatorsequenz einen temperatursensitiven Repressor cI des Phagen λ und einen Promotor umfaßt.

3. Zelle, die eine DNA-Sequenz gemäß Anspruch 1 oder 2 umfaßt und zur Expression der DNA-Sequenz befähigt ist.

4. Zelle gemäß Anspruch 3, wobei der Vektor ein Plasmid ist.

5. Zelle gemäß Anspruch 3 oder 4, wobei der Vektor einen Replikator RSF 1010 umfaßt.

6. Zelle gemäß Anspruch 3, 4 oder 5, wobei der Vektor ein selektierbares Merkergen umfaßt.

7. Zelle gemäß Anspruch 6, wobei das selektierbare Merkergen ein Streptomycin-Resistenz verleihendes Gen ist.

8. Zelle gemäß Anspruch 4, wobei das Plasmid pPRT614 ist.

9. Zelle gemäß einem der Ansprüche 1 bis 8, wobei die Zelle zur.Gattung Escherichia, Serratia, Brevibacterium oder Corynebacterium gehört.

10. Zelle gemäß Anspruch 9, wobei die zu transformierende Zelle Escherichia coli VNIIGenetika TDH-7 ist.

11. Zelle gemäß Anspruch 9, wobei die Zelle Escherichia coli BKIIM B-5318 ist.

12. Verfahren zur Herstellung von L-Threonin, das folgende Schritte umfaßt:
(a) Kultivieren einer Zelle gemäß einem der Ansprüche 1 bis 11 in einem Medium;
(b) Anreichern des L-Threonins in dem Medium und der Zelle;
(c) Gewinnen des L-Threonins aus dem Medium und der Zelle.

## Revendications

1. Cellule d'un procaryote transformée par une séquence d'ADN comprenant
(a) une première séquence opérateur inductible
(b) une deuxième séquence d'ADN directement en aval de la première séquence d'ADN, comprenant un opéron thréonine qui contient une région déficiente en atténuation, où la cellule à transformer est une cellule prototrophe pour l'isoleucine.

2. Cellule selon la revendication 1, où la première séquence opérateur inductible comprend un répresseur cI thermosensible de phage λ et un promoteur.

3. Cellule comprenant une séquence d'ADN selon la revendication 1 ou 2 dans un vecteur capable d'exprimer ladite séquence d'ADN.

4. Cellule selon la revendication 3, où le vecteur est un plasmide.

5. Cellule selon la revendication 3 ou 4, où le vecteur comprend un réplicateur RSF 1010.

6. Cellule selon la revendication 3, 4 ou 5, où le vecteur comprend un gène marqueur sélectionnable.

7. Cellule selon la revendication 6, où ledit gène marqueur sélectif est un gène conférant la résistance à la streptomycine.

8. Cellule selon la revendication 4, où ledit plasmide est pPRT614.

9. Cellule selon l'une quelconque des revendications 1 à 8, où la cellule appartient au genre Escherichia, Serratia, Brevibacterium ou Corynebacterium.

10. Cellule selon la revendication 9, où la cellule transformée est Escherichia coli VNIIGenetika TDH-7.

11. Cellule selon la revendication 9, laquelle est Escherichia coli BKIIM B-5318.

12. Procédé de production de L-thréonine, lequel comprend les étapes consistant à :
(a) cultiver une cellule selon l'une quelconque des revendications 1 à 11 dans un milieu ;
(b) accumuler ladite L-thréonine dans le milieu et la cellule ;
(c) recueillir ladite L-thréonine du milieu et de la cellule.
